# EUROPEAN PATENT SPECIFICATION

(11) **EP 2 756 764 B1**
(45) Date of publication and mention of the grant of the patent: **18.10.2017**
(21) Application number: 12832598.2
(22) Date of filing: 17.09.2012
(51) Int. Cl.: A23L 27/30, A23L 29/30, A23L 33/125, A61K 31/7004

(54) **SWEETENER COMPOSITION FOR PREVENTING AND IMPROVING OBESITY, CONTAINING GLYCOLYSIS INHIBITOR INGREDIENT**
ZUSAMMENSETZUNG ZUR VORBEUGUNG UND LINDERUNG VON ADIPOSITAS MIT EINEM GLYKOLYSEHEMMER-BESTANDTEIL
UTILISATION D'UNE COMPOSITION ÉDULCORANTE CONTENANT UN AGENT INHIBITEUR DE GLYCOLYSE POUR PRÉVENIR ET RÉDUIRE L'OBÉSITÉ

(30) Priority: 15.09.2011 KR 20110092800
(43) Date of publication of application: 23.07.2014
(73) Proprietor: CJ CheilJedang Corporation, Jung-gu Seoul 100-749 (KR)
(72) Inventor: KIM, Young Jae, Seoul 150-072 (KR); PARK, Jin Hee, Gyeonggi-do 411-410 (KR); KIM, Min Hae, Incheon 407-050 (KR); KIM, Seong Bo, Seoul 120-830 (KR); HWANG, Se Hee, Seoul 100-014 (KR); LEE, Young Mi, Gyeonggi-do 420-836 (KR)
(74) Representative: Nevant, Marc
(86) International application number: PCT/KR2012/007424
(87) International publication number: WO 2013/039364

(56) References cited:
- EP-A1- 0 560 284
- EP-A1- 2 111 865
- WO-A1-02/058710
- JP-A- 2005 213 227
- KR-A- 20060 126 999
- KR-A- 20090 077 072
- KR-A- 20100 018 568
- US-A1- 2005 245 459
- US-A1- 2009 304 891

## Description

### Technical Field

The present invention relates to a sweetener composition for use in preventing or treating obesity containing a glucose or D-fructose absorption inhibiting component, a sugar hydrolysis inhibiting sugar or sugar alcohol, and a high sweetness sweetener material as active ingredients.

### Background Art

Sugar contains sucrose as a main ingredient and is one of representative sweeteners exhibiting sweet taste upon adding to food. Sugar has outstanding sweetness and thus has been considered as one of the most preferred sweeteners which are added to various foods and processed foods to improve the food taste and stimulate appetite.

However, recently, as the harmful effects of sugar have been revealed, problems concerning the use thereof have been reported. Specifically, excessive sugar consumption is a major cause of tooth decay as well as various lifestyle related diseases such as obesity, diabetes, and the like. For these reasons, there is global demand for an alternative sweetener as a replacement for sugar.

D-pscicose is an epimer of D-fructose and is a sub-category of functional sugars known as rare sugars. It is known that D-pscicose has a high degree of sweetness equivalent to about 60% to 70% that of sugar and has close to zero calories, and thus is effective in treating adult diseases such as obesity and the like. In addition, D-pscicose is also known to have efficacy to prevent and treat diabetes since D-pscicose is able to inhibit the absorption of sugars such as glucose, D-fructose and the like. Furthermore, D-pscicose is known to have excellent solubility and thus is drawing keep attention for application to foods.

D-pscicose has a relatively good sweetness, but has a relatively lower sweetness than that of sugar. In this regard, the use of D-psicose alone as a sweetener for food additives cannot satisfy consumers accustomed to the taste of sugar, thereby hindering market acceptance. In order to overcome such problems stemming from the use of D-psicose alone, namely, in order to achieve sweetness satisfying general consumers while using D-psicose alone, it is inevitable to increase the amount of D-psicose, which can provide excessive thick feeling to foods utilizing D-psicose, thereby causing deterioration in the texture of foods.

Therefore, there is a strong need for a sweetener applicable to patients suffering from obesity and inhibiting or capable of preventing obesity using D-psicose having low calories and relatively good sweetness.

As the prior art related to the present invention, there are Korea Patent Publication No. 10-2009-0082403A (published on July 30, 2009), Korea Patent Publication No. 10-2011-0035805A (published on April 6, 2011), Korean Patent No. 10-0779160 B1 (published on November 19, 2007), and US patent application No. 2009/304891. US2009304891 discloses a sweetener composition comprising psicose for preventing or treating obesity. Further, a high intensity sweetener and sugar alcohol is disclosed in the same combination with psicose.

### Disclosure

### Technical Problem

The present invention is aimed at providing a sweetener composition for preventing or treating obesity, which has efficacy to prevent or treat obesity while improving quality of sweetness.

### Technical Solution

In accordance with one embodiment of the present invention, a sweetener composition for use in preventing or treating obesity comprises a glucose or D-fructose absorption inhibiting component, a sugar hydrolysis inhibiting sugar or sugar alcohol and a high sweetness sweetener material as active ingredients.

According to the present invention, the glucose and D-fructose absorption inhibiting component is D-psicose; the sugar or sugar alcohol is tagatose; and the high sweetness sweetener material is rebaudioside A.

In accordance with another embodiment of the present invention, the sugar or sugar alcohol may be present in an amount of 0.01 to 200 times the weight of the glucose and D-fructose absorption inhibiting component, and the high sweetness sweetener material may be present in an amount of 0.001 to 2 times the weight of the glucose and D-fructose absorption inhibiting component.

### Advantageous Effects

The present invention provides a sweetener composition that is effective in preventing or treating obesity by utilizing a glucose or D-fructose absorption inhibiting component as defined above and a sugar hydrolysis inhibiting sugar or sugar alcohol as defined above, thereby primarily inhibiting hydrolysis of sugars introduced through foods and secondarily inhibiting absorption of digested glucose and D-fructose, which prevents the intake of a great quantity of sugars.

In addition, the present invention provides a sweetener composition, which includes a glucose or D-fructose absorption inhibiting component as defined above and a sugar hydrolysis inhibiting sugar or sugar alcohol as defined above in a specific ratio, and thus provides outstanding properties in terms of prevention and treatment of obesity as compared with sweetener compositions prepared in component ratios outside the ranges set forth herein.

The sweetener composition for preventing or treating obesity according to the invention further comprises a specific high sweetness sweetener as active ingredient, and can thus reduce the caloric intake while increasing sweetness and has improved quality of sweetness .

According to yet another embodiment, the present invention provides a sweetener composition for preventing or treating obesity, which exhibits outstanding sweetness, thereby avoiding bitter taste, metallic taste or other characteristics of certain high sweetness sweeteners including steviol glycosides while being naturally derived.

### Description of Drawings

Fig. 1 is a graph depicting the change of blood glucose level according to Experimental Example 1.
Fig. 2 is a graph depicting the change of area under the curve of blood glucose according to Experimental Example 2.

### Mode for Invention

Hereinafter, the present invention will be described in more detail. Descriptions of details apparent to those skilled in the art having ordinary knowledge in this technical field or relevant fields will be omitted herein.

The present invention provides a sweetener composition for use in preventing or treating obesity, which contains a glucose or D-fructose absorption inhibiting component, a sugar hydrolysis inhibiting sugar or sugar alcohol, and a high sweetness sweetener material as active ingredients.

According to the present invention the glucose and D-fructose absorption inhibiting component is D-psicose; and the sugar hydrolysis inhibiting sugar or sugar alcohol is tagatose.

Tagatose is a stereoisomer of D-galactose and may be prepared by chemical isomerization of D-galactose using Ca(OH)₂ as a catalyst, or by biotransformation of D-galactose isomerizing D-galactose solutions by an isomerization enzyme.

Tagatose is known as a natural sugar having a high sweetness corresponding to about 90% the sweetness of sucrose and having a low caloric content of about 1.5 kcal per g. Further, tagatose has substantially no side effects and thus is a functional sugar well suited to application to a variety of foods.

The sweetener composition of the invention for use in preventing or treating obesity further includes a high sweetness sweetener material.

The high sweetness sweetener material refers to a sweetener material exhibiting high sweetness several times to hundreds of times higher than that of sugar.

According to the invention, the high sweetness sweetener material is rebaudioside A (Reb A), which is a steviol glycoside. Steviol glycoside refers to a material obtained by processing a water soluble extract of the leaf of *Stevia rebaudiana.* When rebaudioside A is used as a high sweetness sweetener material, the bitter taste, metallic taste and the like characteristic of high sweetness sweeteners such as steviol glycoside may be avoided, thereby providing a sweetener composition for preventing or treating obesity, which has excellent taste.

When rebaudioside A and tagatose are used simultaneously, there is a synergistic effect that improves the quality of sweetness inherent to each substance. For example, although tagatose lacks sweetness persistency, such a drawback of tagatose may be supplemented by rebaudioside A, while the bitterness of rebaudioside A may be overcome by using tagatose. Therefore, the use of tagatose and rebaudioside A in combination may give a bulky effect, which is a heavy feeling of taste in mouth.

According to an embodiment of the present invention, the sweetener composition for use in preventing or treating obesity contains the sugar or sugar alcohol in an amount of 0.01 times to 200 times, preferably 0.01 times to 100 times, more preferably 0.1 times to 50 times the weight of the glucose and D-fructose absorption inhibiting component.

Further, the sweetener composition for use in preventing or treating obesity contains the high sweetness sweetener material in an amount of 0.001 times to 2 times, preferably 0.001 times to 1.5 times, more preferably 0.001 times to 1 time the weight of the glucose and D-fructose absorption inhibiting component.

Within this content range, upon ingesting the sweetener, monosaccharide absorption inhibition in the small intestine and carbohydrate digestion inhibition in the small intestine may be suitably exerted, thereby leading to excellent synergistic effects. With this, sharp rise in blood glucose levels after meals may be significantly lowered and the amount of sugar absorbed into the body may be reduced, thereby indicating excellent effect in improving and/or preventing obesity.

Hereinafter, the present invention will be described in more detail with reference to the following Examples, Comparative Examples and Experimental Examples. These examples are provided for illustration only and are not to be in any way construed as limiting the present invention.

### Examples 1, 2 and Comparative Examples 1 to 3

### Preparation of sweetener composition

Sweetener compositions as listed in Table 1 were prepared.

In Comparative Example 1, 5 g of sugar was used. Sweetener compositions of Comparative Examples 2 and 3 and Examples 1 and 2 were prepared by formulating the components as listed in Table 1 such that the compositions exhibited sweetness similar to that of 5 g of sugar.

**TABLE 1**

| Raw materials (g) | Manufacturer | Relative Sweetness per g of raw material | Com. Ex. 1 | Com. Ex. 2 | Com. Ex. 3 | Ex. 1 | Ex. 2 |
|---|---|---|---|---|---|---|---|
| Sugar | CJ Cheiljedang | 1 | 5 | - | - | - | - |
| Erythritol | Zivogreen | 0.63 | - | 8 | - | - | - |
| D-psicose | CJ Cheiljedang | 0.56 | - | - | 9 | 4 | 2.1 |
| Tagatose | CJ Cheiljedang | 0.92 | - | - | - | 3 | 2 |
| Rebaudioside A | GLG | 200 | - | - | - | - | 0.01 |
| Relative sweetness of sweetener composition | | | 5 | 5.04 | 5.04 | 5 | 5.02 |

Among the sweetener compositions, erythritol used in Comparative Example 2 corresponds to a material that has substantially zero calories and provides only sweetness without affecting blood glucose level. Rebaudioside A used in Example 2 is a steviol glycoside and is a natural high sweetness sweetener prepared by extracting components having sweet taste from steviol glycosides.

### Experimental Example 1

### Measurement of changes in blood glucose level

### (1) Preparation of test specimen for measuring blood glucose and method of intake

In order to measure changes in blood glucose level after consuming each sweetener composition prepared in Comparative Examples 1 to 3 and Examples 1 and 2, the following experiment was carried out for a normal group having a fasting glycemic index of 100 mg/dL or less and consisting of five males and five females in their twenties to forties.

The sweetener composition intake was conducted by providing subjects with an identical meal and then allowing them to drink coffee containing the sweetener composition.

The meals given to the subjects are as listed in Table 2.

**TABLE 2**

| Food materials | Amount used (g) |
|---|---|
| Bread | 75 |
| Ham | 20 |
| Lettuce | 20 |
| Strawberry jam | 20 |
| Crabstick | 30 |
| Cheddar cheese | 10 |

As shown in Table 2, the meal given to the subjects consisted of 75 g of bread, 20 g of lettuce, 20 g of strawberry jam, 30 g of crabstick and 10 g of cheddar cheese. As a result of analysis using Korean food composition table (CanPro 3.0, The Korean Nutrition Society), it was found that the meal had a total caloric content of 356.4 kcal, which consisted of 59.57% of sugar, 18.14% of protein and 22.27% of lipid.

After the meal, the subjects drank coffee made by mixing 1.6 g of sugar free coffee and the sweetener composition prepared in Comparative Examples 1 to 3 and Examples 1 and 2 in 200 g of hot water.

The coffee composition given to the subjects is listed in Table 3.

**TABLE 3**

| Materials (g) | Manufacturer | Comparative Example 1 | Comparative Example 2 | Comparative Example 3 | Example 1 | Example2 |
|---|---|---|---|---|---|---|
| Coffee | Dongsuh Food | 1.6 | 1.6 | 1.6 | 1.6 | 1.6 |
| Sugar | CJ Cheiljedang | 5 | - | - | - | - |
| Erythritol | Zivogreen | - | 8 | - | - | - |
| D-psicose | CJ Cheiljedang | - | - | 9 | 4 | 2.1 |
| Tagatose | CJ Cheiljedang | - | - | - | 3 | 2 |
| Rebaudioside A | GLG | - | - | - | - | 0.01 |

In order to measure changes in blood glucose after the meal, blood glucose level before meal was checked, followed by providing the subjects with the meal and then coffee was given. Blood glucose was measured for 2 hours at intervals of 30 minutes.

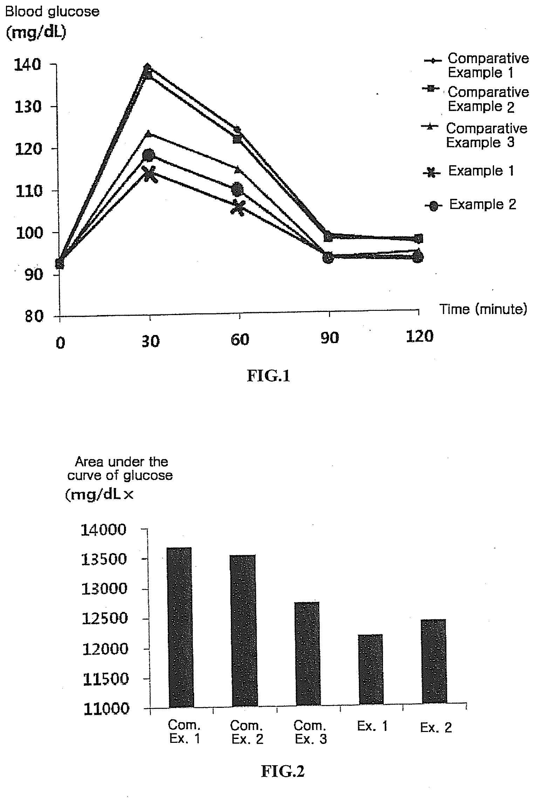

The changes in blood glucose level after meal and coffee intake are summarized in Table 4 (see Fig. 1).

**TABLE 4**

| | Time (min) | 0 (before meal) | 30 | 60 | 90 | 120 |
|---|---|---|---|---|---|---|
| Blood glucose (mg/dL) | Comparative Example 1 | 93.1 | 139.1 | 123.7 | 98.5 | 96.7 |
| | Comparative Example 2 | 92.8 | 137.1 | 121.6 | 97.7 | 97.2 |
| | Comparative Example 3 | 92.4 | 123.3 | 114.5 | 93.1 | 94.1 |
| | Example 1 | 92.9 | *114.3 | *105.5 | *93.4 | 92.8 |
| | Example 2 | 93.2 | *118.3 | *109.5 | *92.9 | 92.4 |

| | | | | | | |
|---|---|---|---|---|---|---|
| * corresponds to time zones in Examples 1 and 2 indicating significant difference of p<0.01 or less in blood glucose as compared with Comparative Example 1 (sugar). | | | | | | |

### Experimental Example 2

### Measurement of changes in area under the curve of blood glucose

The same experiment as in Experimental Example 1 was performed for 2 hours at an interval of 30 minutes to measure G-AUC (Glucose-Area under curve) of the subjects.

The results of G-AUC are summarized in Table 5.

**TABLE 5**

| | Time (min) | 30 | 60 | 90 | 120 |
|---|---|---|---|---|---|
| G-AUC (mg/dL x min) | Comparative Example 1 | 3483 | 7425 | 10758 | 13686 |
| | Comparative Example 2 | 3449 | 7329 | 10619 | 13542 |
| | Comparative Example 3 | 3236 | 6803 | 9917 | 12725 |
| | Example 1 | 3108 | 6405 | 9389 | 12182 |
| | Example 2 | 3173 | 6590 | 9626 | 12405 |

As shown in Table 5, both Examples 1 and 2 showed significant decrease in G-AUC for 120 minutes as compared with Comparative Example 1 (see Fig. 2).

As can be seen from the results of Experimental Examples 1 and 2, an increase in post-meal blood glucose persisted for about 60 minutes and 60 minutes post-meal, the blood glucose level showed a tendency to return to fasting blood glucose level.

When the subjects drank coffee containing the sweetener composition after the meal, the increase in blood glucose 60 minutes after drinking coffee was high in order of Comparative Example 1, Comparative Example 2, Comparative Example 3, Example 2 and Example 1.

Obesity is most likely to occur when the amount of energy absorbed from various components of foods is higher than the energy consumed from activity and is accumulated.

Comparative Example 1 showed the highest increase in blood glucose resulting from the combined increase in blood glucose due to the meal and the coffee containing sugar. Comparative Example 2 showed increase in blood glucose due to meal only.

Comparative Example 3 showed that D-psicose has an effect of lowering increase in blood glucose after the meal through inhibition of monosaccharide absorption in the small intestine.

Examples 1 and 2 appeared to show considerably low absorption of blood glucose as compared with Comparative Examples showing sharp rise in blood glucose after the meal. It appears that such result was caused by rebaudioside A used as the high sweetness sweetener material to increase sweetness, thereby decreasing the amount of tagatose and D-psicose used.

### Experimental Example 3

### Sensory evaluation of sweetener composition

In order to evaluate the sensory perception of the sweetener compositions prepared in Examples 1 and 2, a sensory evaluation test was performed on 25 adult males and females in their twenties to fifties using coffee containing sugar prepared in the same manner as in Experimental Example 1 (corresponding to Comparative Example 1 of Experimental Example 1) and coffee each containing the sweetener composition of Examples 1 and 2.

The sensory analysis was evaluated on a scale of 1 to 5. The results of the sensory analysis are summarized in Table 6.

**TABLE 6**

| | General acceptability | Color acceptability | General flavor acceptability | Taste acceptability | Aftertaste acceptability |
|---|---|---|---|---|---|
| Coffee containing sugar | 3.30 | 3.62 | 3.52 | 3.34 | 3.22 |
| Example 1 | 3.15 | 3.60 | 3.35 | 3.28 | 3.22 |
| Example 2 | 3.32 | 3.55 | 3.50 | 3.35 | 3.25 |

As can be seen from the results of Experimental Examples 1 to 3, it is determined that the sweetener composition of the present invention has excellent synergistic effect of suitably exerted monosaccharide absorption inhibition in the small intestine due to D-psicose and carbohydrate digest inhibition in the small intestine due to tagatose, thereby preventing a sharp increase in blood glucose after a meal while reducing the amount of sugar absorbed into the body. For these reasons, the composition has excellent effects of improving and/or preventing obesity and thus corresponds to the sweetener material for preventing or treating obesity.

## Claims

1. A sweetener composition for use in preventing or treating obesity, said composition comprising, as active ingredients, D-psicose, tagatose and rebaudioside A.

2. The sweetener composition for the use according to claim 1, wherein tagatose is present in an amount of 0.01 times to 200 times the weight of component-psicose, and rebaudioside A is present in an amount of 0.001 to 2 times the weight of component-psicose.

## Patentansprüche

1. Süßstoffzusammensetzung zur Verwendung bei der Vermeidung oder Behandlung von Fettleibigkeit, wobei die Zusammensetzung als aktive Inhaltsstoffe D-Psicose, Tagatose und Rebaudiosid A umfasst.

2. Süßstoffzusammensetzung zur Verwendung nach Anspruch 1, wobei Tagatose in einer Menge des 0,01-fachen bis 200-fachen des Gewichts der Psicose-Komponente vorhanden ist und Rebaudiosid A in einer Menge des 0,001- bis 2-fachen des Gewichts der Psicose-Komponente vorhanden ist.

## Revendications

1. Composition d'édulcorant pour une utilisation dans la prévention ou le traitement de l'obésité, ladite composition comprenant, comme ingrédients actifs, du D-psicose, du tagatose et du rébaudioside A.

2. Composition d'édulcorant pour l'utilisation selon la revendication 1, dans laquelle le tagatose est présent dans une quantité de 0,01 fois à 200 fois la masse du constituant psicose, et le rébaudioside A est présent dans une quantité de 0,001 à 2 fois la masse du constituant psicose.
